# EUROPEAN PATENT APPLICATION

(11) **EP 4 675 006 A2**
(43) Date of publication of application: **07.01.2026**
(21) Application number: 25217423.0
(22) Date of filing: 24.04.2020
(51) Int. Cl.: C23C 16/40

(54) **COATED DRUG COMPOSITIONS AND METHODS OF PREPARING THE SAME**

(30) Priority: 26.04.2019 US 201962839285 P
(62) Divisional of application: 20794239.2
(71) Applicant: Applied Materials, Inc., Santa Clara, CA 95054-3299 (US)
(72) Inventor: WANG, Fei, Fremont, 94539 (US); NEIKIRK, Colin C., Mountain View, 94040 (US); FRANKEL, Jonathan, Los Gatos, 95030 (US); NARWANKAR, Pravin K., Sunnyvale, 94086 (US)
(74) Representative: Zimmermann & Partner Patentanwälte mbB

(57) **Abstract**

A method of preparing a pharmaceutical composition is provided. The method includes the deposition of a metal oxide layer(s) followed byapplying a polymer layer(s). This produces a pharmaceutical composition comprising a drug containing core enclosed by one or more metal oxide materials and one more polymer materials.

## Description

### TECHNICAL FIELD

This disclosure pertains to coated drug compositions and methods of preparing coated drug compositions.

### BACKGROUND

It is of great interest to the pharmaceutical industry to develop improved formulations of active pharmaceutical ingredients (API). Formulation can influence the stability and bioavailability of the API as well as other characteristics. Formulation can also influence various aspects of drug product (DP) manufacture, for example, ease and safety of the manufacturing process.

Numerous technologies for encapsulating or coating both API and DP have been developed, e.g., polymer mesh coating, pan coating, aerosolized coating, fluidized bed reactor coating and atomic layer deposition coating.

### SUMMARY

The invention is set out in the appended set of claims.

In one aspect, a method of preparing coated particle having an active pharmaceutical ingredient (API)-containing core enclosed by one or more metal oxide layers or inorganic oxide layers and one or more polymer layers is provided. A metal oxide layer is applied to the core first by atomic layer deposition (ALD). After the metal oxide layer is deposited, a polymer layer is applied by molecular layer deposition (MLD) or hybrid ALD/MLD deposition. These steps can be repeated to create particles having an API-containing core enclosed by multiple layers (e.g., multiple alternating layers) of metal oxide and polymer. The methods can be applied to particles of API or particles comprising one more APIs and one or more excipients.

The metal oxide layer is thin and conforms to the API-containing core. This layer can greatly improve the flowability and other handling characteristics of the API-containing core. It can also alter the stability and dissolution rate of the API. The polymer layer can adjust the dissolution rate of the API, such as for an extended release coating an enteric coating and can increase the stability of the API, e.g., can increase resistance to oxidation and/or alteration in crystalline form. Thus, the coating layers can reduce transition of the API form an amorphous form to a crystalline form. The coating layers can provide bulking and/or improve compressibility thereby reducing the need for excipients. By reducing the need for additional excipients, the coated particles can be used to create dosage forms with high drug loading.

Described herein is a composition comprising individual particles comprising an API-containing core enclosed by an inner metal oxide layer and polymer layer adjacent the metal oxide layer, wherein the core has a median particle size, on a volume average basis, between 0.1 µm and 10 or 20 µm, the inner metal oxide layer has an average thickness of 0.01 or 0.1 nm to 30 nm and the polymer layer has an average thickness of 0.1 nm to 400 nm.

In various embodiments: the inner metal oxide layer conforms to the core and is substantially pin-hole free and the outer polymer layer conforms to the inner metal oxide layer and is substantially pin-hole free; the core has a median particle size, on a volume average basis between 0.1 µm and 10 or 20 µm; the core further comprises one or more pharmaceutically acceptable excipients; the core comprises a first API and a second API; the core consists of an API; the core comprises a first API and a second API; the particles comprise an outer metal oxide layer adjacent the polymer layer; the metal oxide layer comprises aluminum oxide, titanium oxide, iron oxide, silicon dioxide, magnesium oxide or zinc oxide; the polymer layer comprises a polyamide, a polyimide, a polyurea, a polyurethane, a polythiourea, a polyester or a polyimine.

Also described herein is pharmaceutical composition comprising coated particles comprising an API-containing core enclosed by one or more metal oxide layers and one or more polymer layers, prepared by a method comprising the sequential steps of: (a) performing atomic layer deposition to apply a metal oxide layer to particles comprising an API thereby preparing particles comprising an API enclosed by a metal oxide layer; and subsequently; and (b) performing molecular layer deposition to apply a polymer layer to the particles comprising a drug enclosed by a metal oxide layers, thereby preparing coated particles comprising an API-containing core enclosed by one or more metal oxide layers and one or more polymer layers.

In various embodiments: the step of performing atomic layer deposition comprises:
(a1) loading the particles comprising the API into a reactor; (a2) applying a vaporous or gaseous metal precursor to the particles in the reactor; (a3) performing one or more pump-purge cycles of the reactor using inert gas; (a4) applying a vaporous or gaseous oxidant to the particles in the reactor; and (a5) performing one or more pump-purge cycles of the reactor using inert gas.

In various embodiments: steps (a2) - (a5) are performed two or more times to increase the total thickness of the metal oxide layer before step (b) is performed; steps (a2) - (a5) are performed 5 - 50 times to increase the total thickness of the metal oxide layer before step (b) is performed;

In various embodiments: the step of performing molecular layer deposition comprises: (b1) applying vaporous polymer precursor A to the particles comprising an API containing enclosed by a oxide layer in the reactor; (b2) performing one or more pump-purge cycles of the reactor using inert gas; (b3) applying vaporous polymer precursor B to the particles in the reactor; and (b4) performing one or more pump-purge cycles of the reactor using inert gas.

In various embodiments: steps (b1) - (b4) are performed two or more times to increase the total thickness of the polymer layer; steps (a2) - (a5) are performed 5 - 50 times to increase the total thickness of the metal oxide layer before step (b) is performed; the reactor contents are agitated prior to and/or during step (a) and/or step (b); the reactor pressure is allowed to stabilize following step (a1), step (a2), and/or step (a4); the reactor contents are agitated prior to and/or during step (a1), step (a3), and/or step (a5); a subset of vapor or gaseous content is pumped out prior to step (a3) and/or step (a5); the metal oxide layer has a thickness in range of 0.01 or 0.1 nm to 20 or 30 nm; the polymer layer has a thickness in range of 0.1 nm to 100 nm; steps (a2) - (a5) are repeated two or more times and the particles are not removed from the reactor between each repetition; steps (a2) - (a5) are repeated two or more times and the particles are not removed from the reactor during step (a); steps (b1) - (b4) are repeated two or more times and the particles are not removed from the reactor between each repetition; steps (b1) - (b4) are repeated two or more times and the particles are not removed from the reactor during step (b); the particles comprising an API further comprise one or more pharmaceutically acceptable excipients; the particles comprising an API have a median particle size, on a volume average basis between 0.1 µm and 10 or 20 µm prior to step (a); the particles subjected to step (a) comprise a first API and a second API; the particles subjected to step (a) consist of an API; the particles subjected to step (a) consist of a first API and a second API; the particles subjected to step (a) comprise an API and one or more pharmaceutically acceptable excipients; the particles subjected to step (a) comprise a first API, a second API and one or more pharmaceutically acceptable excipients; the particles subjected to step (a) consist of an API and one or more pharmaceutically acceptable excipients; the particles subjected to step (a) consist of a first API, a second API and one or more pharmaceutically acceptable excipients; the metal oxide layer comprises aluminum oxide, titanium oxide, iron oxide, silicon dioxide, magnesium oxide or zinc oxide; the polymer layer comprises a polyamide, a polyimide, a polyurea, a polyurethane, a polythiourea, a polyester or a polyimine; the polymer layer is biodegradable; the composition comprises an inner metal oxide layer, an intermediate polymer layer and an out metal oxide layer.

Also described herein is a method of preparing coated particles comprising an active pharmaceutical ingredient (API)-containing core enclosed by one or more metal oxide layers and one or more polymer layers, the method comprising the sequential steps of: (a) performing atomic layer deposition to apply a metal oxide layer to particles comprising an API thereby preparing particles comprising an API enclosed by a metal oxide layer; and subsequently; (b) performing molecular layer deposition to apply a polymer layer to the particles comprising a drug enclosed by a metal oxide layer, thereby preparing coated particles comprising an API-containing core enclosed by one more metal oxide layers and one or more polymer layers.

In various embodiments: the step of performing atomic layer deposition comprises: (a1) loading the particles comprising the API into a reactor; (a2) applying a vaporous or gaseous metal precursor to the particles in the reactor; (a3) performing one or more pump-purge cycles of the reactor using inert gas; (a4) applying a vaporous or gaseous oxidant to the particles in the reactor; and (a5) performing one or more pump-purge cycles of the reactor using inert gas.

In various embodiments: steps (a2) - (a5) are performed two or more times to increase the total thickness of the metal oxide layer before step (b) is performed; steps (a2) - (a5) are performed 5 - 50 times to increase the total thickness of the metal oxide layer before step (b) is performed.

In various embodiments: the step of performing molecular layer deposition comprises: (b1) applying vaporous polymer precursor A to the particles comprising an API containing enclosed by a metal oxide layer in the reactor; (b2) performing one or more pump-purge cycles of the reactor using inert gas; (b3) applying vaporous polymer precursor B to the particles in the reactor; and (b4) performing one or more pump-purge cycles of the reactor using inert gas.

In various embodiments: steps (b1) - (b4) are performed two or more times to increase the total thickness of the polymer layer; steps (a2) - (a5) are performed 5 - 50 times to increase the total thickness of the metal oxide layer before step (b) is performed; the reactor contents are agitated prior to and/or during step (a) and/or step (b); the reactor pressure is allowed to stabilize following step (a1), step (a2), and/or step (a4); reactor contents are agitated prior to and/or during step (a1), step (a3), and/or step (a5); a subset of vapor or gaseous content is pumped out prior to step (a3) and/or step (a5); the metal oxide layer has a thickness in range of 0.01 or 0.1 nm to 10 or 20 nm; the polymer layer has a thickness in range of 0.1 nm to 400 nm; steps (a2) - (a5) are repeated two or more times and the particles are not removed from the reactor between each repetition; steps (a2) - (a5) are repeated two or more times and the particles are not removed from the reactor during step (a); steps (b1) - (b4) are repeated two or more times and the particles are not removed from the reactor between each repetition; steps (b1) - (b4) are repeated two or more times and the particles are not removed from the reactor during step (b); the particles comprising an API further comprise one or more pharmaceutically acceptable excipients; the particles comprising an API have a median particle size, on a volume average basis between 0.1 µm and 20 µm prior to step (a); the particles have a median particle size, on a volume average basis between 0.1 µm and 5 or 10 µm prior to step (a); the particles have a median particle size, on a volume average basis between 0.1 µm and 1000 µm prior to step (a); the particles subjected to step (a) comprise a first API and a second API; the particles subjected to step (a) consist of an API; the particles subjected to step (a) consist of a first API and a second API; the particles subjected to step (a) comprise an API and one or more pharmaceutically acceptable excipients; the particles subjected to step (a) comprise a first API, a second API and one or more pharmaceutically acceptable excipients; the particles subjected to step (a) consist of an API and one or more pharmaceutically acceptable excipients; the particles subjected to step (a) consist of a first API, a second API and one or more pharmaceutically acceptable excipients; the method comprises admixing the coated particles comprising an API-containing core enclosed by one or more metal oxide layers and one or more polymer layers with a pharmaceutically acceptable diluent or carrier; the method further comprises processing the coated particles comprising an API-containing core enclosed by one or more metal oxide layers and one or more polymer layers to form a tablet or capsule; the method further comprises admixing the coated particles comprising an API-containing core enclosed by one or more metal oxide layers and one or more polymer layers with a pharmaceutically acceptable diluent or carrier to form a mixture a processing the mixture to form a table or capsule; the method further comprises performing atomic layer deposition to apply a metal oxide layer to the particles comprising an API-containing core enclosed by a metal oxide layer and a polymer layer thereby preparing particles comprising an API enclosed by an inner metal oxide layer, an intermediate polymer layer and an outer metal oxide layer; the method further comprises performing molecular layer deposition to apply a polymer layer to the particles comprising an API enclosed by an inner metal oxide layer, an intermediate polymer layer and an outer metal oxide layer thereby preparing particles comprising an API enclosed by an inner metal oxide layer, an inner intermediate polymer layer and an outer intermediate metal oxide layer and an outer polymer layer; the method further comprises admixing the coated particles comprising an API-containing core enclosed by one or more metal oxide layers and one or more polymer layers with a pharmaceutically acceptable diluent or carrier; the method further comprises processing the coated particles comprising an API-containing core enclosed by one or more metal oxide layers and one or more polymer layers to form a tablet or capsule; and the method further comprises admixing the coated particles comprising an API-containing core enclosed by one or more metal oxide layers and one or more polymer layers with a pharmaceutically acceptable diluent or carrier to form a mixture a processing the mixture to form a table or capsule.

Thus, the metal oxide deposition method includes the sequential steps of (a1) loading the particles of API or particles comprising API into a reactor, (a2) applying a vaporous or gaseous metal precursor to the particles in the reactor, (a3) performing one or more pump-purge cycles of the reactor using inert gas, (a4) applying a vaporous or gaseous oxidant to the particles in the reactor, and (a5) performing one or more pump-purge cycles of the reactor using inert gas. This produces a pharmaceutical composition comprising an API containing core enclosed by one or more metal oxide materials. The metal oxide coated particles are subsequently coated with a polymer. Thus, the polymer deposition method includes the sequential steps of (b1) applying a vaporous or gaseous precursor A to the particles in the reactor, (b2) performing one or more pump-purge cycles of the reactor using inert gas, (b3) applying a vaporous or gaseous precursor B to the particles in the reactor, and (b4) performing one or more pump-purge cycles of the reactor using inert gas. Precursor A and precursor B react and can form a polymer of the ABAB type. Other types of polymers can be formed using other precursors. For example, precursor A, precursor B and precursor C.

The polymer layer can be a hybrid layer. The hybrid polymer deposition method includes the sequential steps of: (b1') applying a vaporous or gaseous precursor A to the particles in the reactor, (b2') performing one or more pump-purge cycles of the reactor using inert gas, (b3') applying a vaporous or gaseous metal oxide precursor to the particles in the reactor, and (b4') performing one or more pump-purge cycles of the reactor using inert gas.

In various embodiments: the temperature of the interior of the reactor need not exceed 100°C, 50°C, 40°C; one or more metal oxide materials may include aluminum oxide, titanium oxide, iron oxide, gallium oxide, magnesium oxide, silicon dioxide,zinc oxide, niobium oxide, hafnium oxide, tantalum oxide, lanthanum oxide, and/or zirconium dioxide; one or more metal oxide materials may consist of aluminum oxide and/or titanium oxide; the oxidant may be selected from the group of water, ozone, and organic peroxide; the one or more polymer layers may include polymer amide, polyester, polyurethane, polyurea, polyimide; and the MLD precursors may be selected from any organic molecule which contains difunctional group, such as diol, diamine, diisocyanate, dichloride, dialdehyde.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

Other features and advantages of the invention will be apparent from the following detailed description and figures, and from the claims.

### BRIEF DESCRIPTION OF THE DRAWINGS

FIG. 1 is a schematic illustration of a rotary reactor for ALD and/or CVD coating of particles, e.g., particles of an API or particles that comprise an API and an excipient.
FIG. 2 is a schematic illustration of an MLD coating process.
FIG. 3 is an SEM image of a coated API particle and a TEM cross section of a coated API particle.
FIG. 4 is a graph depicting the dissolution of uncoated acetaminophen particles, metal oxide coated acetaminophen particles and acetaminophen particle coated with both a metal oxide layer and a polymer (polyamide) layer.
FIG. 5 is TEM image of a cross-section of an API particle coated with alternating metal oxide and polyamide coating layers.

### DETAILED DESCRIPTION

There are various methods for encapsulating API particles in a polymer, e.g., hot melt extrusion. In many cases, these methods result in a polymer coating that is relatively thick. While such polymer coatings can impart desirable properties, the high ratio of polymer to API can make it difficult to create a drug product in which the volume fraction of API is as high as desired. In addition, the layer of polymer encapsulating the API can be non-uniform, making it difficult to provide formulations with consistent properties. There is a continuing need for improved coating technologies that can impart desirable characteristics to both active pharmaceutical ingredients and drug products.

The present disclosure provides methods of preparing pharmaceutical compositions comprising API (drug) containing particles encapsulated by one or more layers of metal oxide and one or more layers of a polymer. The coating layers are conformal and of controlled thickness from several nanometers to several micrometers in total. The articles to be coated can be composed of only API or a combination of API and one or more excipients. The coating process described herein can provide an API with an increased glass transition temperature for the API relative to uncoated API, a decreased rate of crystallization for an amorphous form of the API relative to uncoated API, and decreased surface mobility of API molecules in the particle compared to uncoated API. Importantly, API particle dissolution can be altered through control conformal polymer layer thickness, composition and functional group. Because the coating is relatively thin, drug products with high drug loading can be achieved. Finally, there are benefits with respect to cost and ease of manufacture because multiple coatings can be applied in the same reactor.

The term "drug" in its broadest sense includes all small molecule (e.g., non-biologic) APIs. The drug could be selected from the group consisting of an analgesic, an anesthetic, an anti-inflammatory agent, an anthelmintic, an anti-arrhythmic agent, an antiasthma agent, an antibiotic, an anticancer agent, an anticoagulant, an antidepressant, an antidiabetic agent, an antiepileptic, an antihistamine, an antitussive, an antihypertensive agent, an antimuscarinic agent, an antimycobacterial agent, an antineoplastic agent, an antioxidant agent, an antipyretic, an immunosuppressant, an immunostimulant, an antithyroid agent, an antiviral agent, an anxiolytic sedative, a hypnotic, a neuroleptic, an astringent, a bacteriostatic agent, a beta-adrenoceptor blocking agent, a blood product, a blood substitute, a bronchodilator, a buffering agent, a cardiac inotropic agent, a chemotherapeutic, a contrast media, a corticosteroid, a cough suppressant, an expectorant, a mucolytic, a diuretic, a dopaminergic, an antiparkinsonian agent, a free radical scavenging agent, a growth factor, a haemostatic, an immunological agent, a lipid regulating agent, a muscle relaxant, a parasympathomimetic, a parathyroid calcitonin, a biphosphonate, a prostaglandin, a radio-pharmaceutical, a hormone, a sex hormone, an anti-allergic agent, an appetite stimulant, an anoretic, a steroid, a sympathomimetic, a thyroid agent, a vaccine, a vasodilator and a xanthine.

Exemplary types of small molecule drugs include, but are not limited to, acetaminophen, clarithromycin, azithromycin, ibuprofen, fluticasone propionate, salmeterol, pazopanib HCl, palbociclib, and amoxicillin potassium clavulanate.

The term "metal oxide material," in its broadest sense includes all materials formed from the reaction of elements considered metals with oxygen-based oxidants. Exemplary metal oxide materials include, but are not limited to, aluminum oxide, titanium dioxide, iron oxide, gallium oxide, magnesium oxide, zinc oxide, niobium oxide, hafnium oxide, tantalum oxide, lanthanum oxide, and zirconium dioxide. Exemplary oxidants include, but are not limited to, water, ozone, and inorganic peroxide.

Atomic layer deposition is a thin film deposition technique in which the sequential addition of self-limiting monolayers of an element or compound allows deposition of a film with thickness and uniformity controlled to the level of an atomic or molecular monolayer. Self-limited means that only a single atomic layer is formed at a time, and a subsequent process step is required to regenerate the surface and allow further deposition.

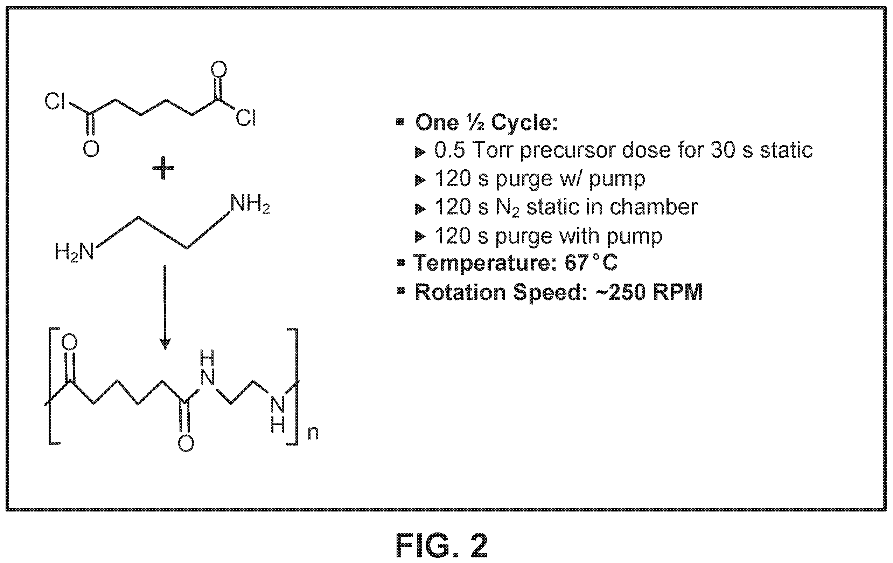

Molecular Layer Deposition is analogous to Atomic Layer Deposition but using organic precursors and forming completely organic thin films. During a typical MLD process two homo-bifunctional precursors are used (FIG. 2). A first precursor is introduced into a chamber. The molecules of the first precursor react with reactive groups on the substrate surface via the corresponding linking chemistry to add a molecular layer of the first precursor on the substrate surface with new reactive sites. After purging, a second precursor is introduced and the molecules of the second precursor react with the new reactive sites provided by the first precursor generating a molecular layer of the first precursor linked to the second precursor. This is followed by another purse cycle. Using adipoyl chloride (AC) and 1,6-hexanediamine (HD) as precursors, the chemistry can be depicted as follows:
First precursor reaction

   |-NH₂ + ClCO(CH₂)₄COCl (g) →

   |-NHCO(CH₂ )₄COCl + HCl (g)
Second precursor reaction

   |-NHCO(CH₂)₄COC + H₂ N(CH₂)₆NH₂ (g) →

   |-NHCO(CH₂)₄CO-NH(CH₂)₆NH₂ + HCl (g)

Succinyl chloride and 1,3-diaminopropane are another suitable first and second precursor pair.

MLD can be used to create a variety of biocompatible polymer coatings, including: hydrophobic polymer coatings (e.g., Poly(caprolactone), Poly(lactic acid). Poly(glycolide) and copolymers thereof); hydrophilic Polymers (e.g, Poly(ether esters)); and pH responsive polymers (e.g., Poly(ether amides) and Poly(ether esters) with carboxylic acid pendant groups). Precursors which can be used in the first precursor reaction include: acid chlorides (e.g., Succinyl chloride, Glutaryl chloride, and Adipoyl chloride). Precursors which can be used in the second precursor reaction include: diols (e.g., Diethylene glycol); hydrophobic diamines (e.g., Ethylene diamine); hydrophilic diamines (2,2'-(Ethylenedioxy)bis(ethylamine)). Crosslinking Acid Chlorides (e.g., Trimesoyl chloride (1,3,5-Benzenetricarbonyl trichloride) can also be incorporated.

Hybrid MLD/ALD Layer Deposition is analogous to atomic layer deposition and molecular layer deposition, using one MLD precursor and one metal oxide precursor and forming a hybrid layer with both organic inorganic composition. During a typical MLD/ALD hybrid process two homo-bifunctional precursors are used. A first precursor is introduced into a chamber. The molecules of the first precursor react with reactive groups on the substrate surface via the corresponding linking chemistry to add a molecular layer of the first precursor on the substrate surface with new reactive sites. After purging, a second precursor, which is a metal oxide precursor, is introduced and the molecules of the second precursor react with the new reactive sites provided by the first precursor generating hybrid layer composed of the first precursor linked to the second precursor. This is followed by another purge cycle. Using TMA and 1,2 ethylanediol as precursors, the chemistry can be depicted as follows:

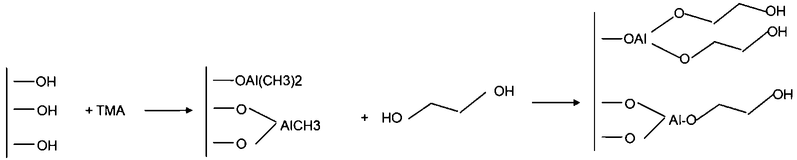

Other pairs of useful precursors include: succinyl chloride adnd 1,3-diaminopropane

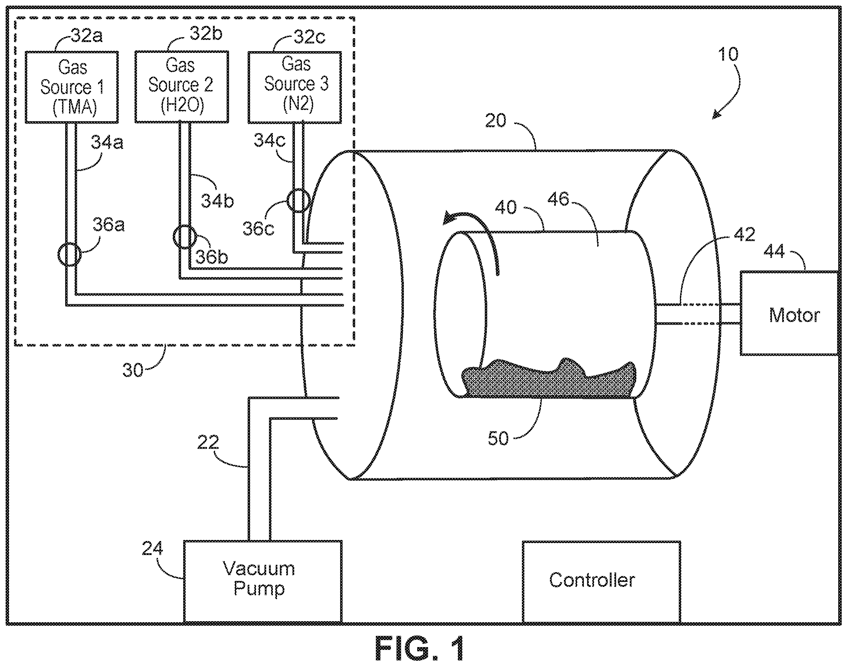

The term "reactor system" in its broadest sense includes all systems that could be used to perform ALD or mixed ALD/CVD or CVD. An exemplary reactor system is illustrated in FIG. 1 and further described below in the context of an ALD reaction. The same or a similar reactor system can be used to perform MLD, hybrid MLD/ALD.

FIG. 1 illustrates a reactor system 10 for performing coating of particles, with thin-film coatings. The reactor system 10 can perform the coating using ALD and/or hybrid, and/or MLD coating conditions. The reactor system 10 permits a coating process (ALD or MLD or hybrid), to be performed at higher (above 50 °C, e.g., 50-100 °C) or lower processing temperature, e.g., below 50 °C, e.g., at or below 35 °C. For example, the reactor system 10 can form thin-film metal oxides on the particles primarily by ALD at temperatures of 22-35 °C, e.g., 25-35 °C, 25-30 °C, or 30-35 °C. In general, the particles can remain or be maintained at such temperatures. This can be achieved by having the reactant gases and/or the interior surfaces of the reactor chamber (e.g., the chamber 20 and drum 40 discussed below) remain or be maintained at such temperatures.

Again, illustrating an ALD process, the reactor system 10 includes a stationary vacuum chamber 20 which is coupled to a vacuum pump 24 by vacuum tubing 22. The vacuum pump 24 can be an industrial vacuum pump sufficient to establish pressures less than 1 Torr, e.g., 1 to 100 mTorr, e.g., 50 mTorr. The vacuum pump 24 permits the chamber 20 to be maintained at a desired pressure, and permits removal of reaction byproducts and unreacted process gases.

In operation, the reactor 10 performs the ALD thin-film coating process by introducing gaseous precursors of the coating into the chamber 20. The gaseous precursors are spiked alternatively into the reactor. This permits the ALD process to be a solvent-free process. The half-reactions of the ALD process are self-limiting, which can provide Angstrom level control of deposition. In addition, the ALD reaction can be performed at low temperature conditions, such as below 50 °C, e.g., below 35 °C.

The chamber 20 is also coupled to a chemical delivery system 30. The chemical delivery system 20 includes three or more gas sources 32a, 32b, 32c coupled by respective delivery tubes 34a, 34b, 34c and controllable valves 36a, 36b, 36c to the vacuum chamber 20. The chemical delivery system 30 can include a combination of restrictors, gas flow controllers, pressure transducers, and ultrasonic flow meters to provide controllable flow rate of the various gasses into the chamber 20. The chemical delivery system 30 can also include one or more temperature control components, e.g., a heat exchanger, resistive heater, heat lamp, etc., to heat or cool the various gasses before they flow into the chamber 20. Although FIG. 1 illustrates separate gas lines extending in parallel to the chamber for each gas source, two or more of the gas lines could be joined, e.g., by one or more three-way valves, before the combined line reaches the chamber 20. In addition, although FIG. 1 illustrates three gas sources, the use of four gas sources could enable the in-situ formation of laminate structures having alternating layers of two different metal oxides.

Two of the gas sources provide two chemically different gaseous reactants for the coating process to the chamber 20. Suitable reactants for ALD methods include any of or a combination of the following: monomer vapor, metal-organics, metal halides, oxidants, such as ozone or water vapor, and polymer or nanoparticle aerosol (dry or wet). For example, the first gas source 32a can provide gaseous trimethylaluminum (TMA) or titanium tetrachloride (TiCl₄), whereas the second gas source 32b can provide water vapor.

One of the gas sources can provide a purge gas. In particular, the third gas source can provide a gas that is chemically inert to the reactants, the coating, and the particles being processed. For example, the purge gas can be N₂, or a noble gas, such as argon.

A rotatable coating drum 40 is held inside the chamber 20. The drum 40 can be connected by a drive shaft 42 that extends through a sealed port in a side wall of the chamber 20 to a motor 44. The motor 44 can rotate the drum at speeds of 1 to 100 rpm. Alternatively, the drum can be directly connected to a vacuum source through a rotary union.

The particles to be coated, shown as a particle bed 50, are placed in an interior volume 46 of the drum 40. The drum 40 and chamber 20 can include sealable ports (not illustrated) to permit the particles to be placed into and removed from the drum 40.

The body of the drum 40 is provided by one or more of a porous material, a solid metal, and a perforated metal. The pores through the cylindrical side walls of the drum 40 can have a dimension of 10 µm.

In operation, one of the gasses flows into chamber 20 from the chemical delivery system 30 as the drum 40 rotates. A combination of pores (1-100 um), holes (0.1-10 mm), or large openings in the coating drum serve to confine the particles in the coating drum while allowing rapid delivery of precursor chemistry and pumping of byproducts or unreacted species. Due to the pores in the drum 40, the gas can flow between the exterior of the drum 40, i.e., the reactor chamber 20, and the interior of the drum 40. In addition, rotation of the drum 40 agitates the particles to keep them separate, ensuring a large surface area of the particles remains exposed. This permits fast, uniform interaction of the particle surface with the process gas.

In some implementations, one or more temperature control components are integrated into the drum 40 to permit control of the temperature of the drum 40. For example, resistive heater, a thermoelectric cooler, or other component can in or on the side walls of the drum 40.

The reactor system 10 also includes a controller 60 coupled to the various controllable components, e.g., vacuum pump 24, gas distribution system 30, motor 44, a temperature control system, etc., to control operation of the reactor system 10. The controller 60 can also be coupled to various sensors, e.g., pressure sensors, flow meters, etc., to provide closed loop control of the pressure of the gasses in the chamber 20.

In general, the controller 60 can operate the reactor system 10 in accord with a "recipe." The recipe specifies an operating value for each controllable element as a function of time. For example, the recipe can specify the times during which the vacuum pump 24 is to operate, the times of and flow rate for each gas source 32a, 32b, 32c, the rotation rate of the motor 44, etc. The controller 60 can receive the recipe as computer-readable data (e.g., that is stored on a non-transitory computer readable medium).

The controller 60 and other computing devices part of systems described herein can be implemented in digital electronic circuitry, or in computer software, firmware, or hardware. For example, the controller can include a processor to execute a computer program as stored in a computer program product, e.g., in a non-transitory machine readable storage medium. Such a computer program (also known as a program, software, software application, or code) can be written in any form of programming language, including compiled or interpreted languages, and it can be deployed in any form, including as a standalone program or as a module, component, subroutine, or other unit suitable for use in a computing environment. In some implementations, the controller 60 is a general purpose programmable computer. In some implementations, the controller can be implemented using special purpose logic circuitry, e.g., an FPGA (field programmable gate array) or an ASIC (application specific integrated circuit).

### Operation

The operation is illustrated with an ALD process, but the operation is similar for MLD, or hybrid. Initially, particles are loaded into the drum 40 in the reactor system 10. The particles can have a solid core comprising a drug, e.g., one of the drugs discussed above. Once any access ports are sealed, the controller 60 operates the reactor system 10 according to the recipe in order to form the thin-film metal oxide layers on the particles.

In particular, the two reactant gases are alternately supplied to the chamber 20, with each step of supplying a reactant gas followed by a purge cycle in which the inert gas is supplied to the chamber 20 to force out the reactant gas and by-products used in the prior step. Moreover, one or more of the gases (e.g., the reactant gases and/or the inert gas) can be supplied in pulses in which the chamber 20 is filled with the gas to a specified pressure, a delay time is permitted to pass, and the chamber is evacuated by the vacuum pump 24 before the next pulse commences.

In particular, the controller 60 can operate the reactor system 10 as follows.

In a first reactant half-cycle, while the motor 44 rotates the drum 40 to agitate the particles 50:
i) The gas distribution system 30 is operated to flow the first reactant gas, e.g., TMA, from the source 32a into the chamber 20 until a first specified pressure is achieved. The specified pressure can be 0.1 Torr to half of the saturation pressure of the reactant gas.
ii) Flow of the first reactant is halted, and a specified delay time is permitted to pass, e.g., as measured by a timer in the controller. This permits the first reactant to flow through the particle bed in the drum 40 and react with the surface of the particles 50 inside the drum 40.
iii) The vacuum pump 50 evacuates the chamber 20, e.g., down to pressures below 1 Torr, e.g., to 1 to 100 mTorr, e.g., 50 mTorr.

These steps (i)-(iii) can be repeated a number of times set by the recipe, e.g., two to ten times, e.g., six times.

Next, in a first purge cycle, while the motor 44 rotates the drum to agitate the particles 50:
iv) The gas distribution system 30 is operated to flow the inert gas, e.g., N₂, from the source 32c into the chamber 20 until a second specified pressure is achieved. The second specified pressure can be 1 to 100 Torr.
v) Flow of the inert gas is halted, and a specified delay time is permitted to pass, e.g., as measured by the timer in the controller. This permits the inert gas to flow through the pores in the drum 40 and diffuse through the particles 50 to displace the reactant gas and any vaporous by-products.
vi) The vacuum pump 50 evacuates the chamber 20, e.g., down to pressures below 1 Torr, e.g., to 1 to 500 mTorr, e.g., 50 mTorr.

These steps (iv)-(vi) can be repeated a number of times set by the recipe, e.g., six to twenty times, e.g., sixteen times.

In a second reactant half-cycle, while the motor 44 rotates the drum 40 to agitate the particles 50:
vii) The gas distribution system 30 is operated to flow the second reactant gas, e.g., H2O, from the source 32a into the chamber 20 until a third specified pressure is achieved. The third pressure can be 0.1 Torr to half of the saturation pressure of the reactant gas.
viii) Flow of the second reactant is halted, and a specified delay time is permitted to pass, e.g., as measured by the timer in the controller. This permits the second reactant to flow through the pores in the drum 40 and react with the surface of the particles 50 inside the drum 40.
ix) The vacuum pump 50 evacuates the chamber 20, e.g., down to pressures below 1 Torr, e.g., to 1 to 500 mTorr, e.g., 50 mTorr.

These steps (vii)-(ix) can be repeated a number of times set by the recipe, e.g., two to ten times, e.g., six times.

Next, a second purge cycle is performed. This second purge cycle can be identical to the first purge cycle, or can have a different number of repetitions of the steps (iv)-(vi) and/or different delay time and/or different pressure.

The cycle of the first reactant half-cycle, first purge cycle, second reactant half cycle and second purge cycle can be repeated a number of times set by the recipe, e.g., one to ten times.

As noted above, the coating process can be performed at low processing temperature, e.g., below 50 °C, e.g., at or below 35 °C. In particular, the particles can remain or be maintained at such temperatures during all of steps (i)-(ix) noted above. In general, the temperature of the interior of the reactor chamber does not exceed 35°C during of steps (i)-(ix). This can be achieved by having the first reactant gas, second reactant gas and inert gas be injected into the chamber at such temperatures during the respective cycles. In addition, physical components of the chamber of the chamber can remain or be maintained at such temperatures, e.g., using a cooling system, e.g., a thermoelectric cooler, if necessary.

### Process for Preparing Pharmaceutical Compositions Comprising Drugs Encapsulated by One or More Layers of Metal Oxide

Provided are two exemplary methods for a pharmaceutical composition comprising a drug-containing core enclosed by one or more metal oxide materials. The first exemplary method includes the sequential steps of: (a) loading the particles comprising the drug into a reactor, (b) applying a vaporous or gaseous metal precursor to the substrate in the reactor, (c) performing one or more pump-purge cycles of the reactor using inert gas, (d) applying a vaporous or gaseous oxidant to the substrate in the reactor, and (e) performing one or more pump-purge cycles of the reactor using inert gas. In some embodiments of the first exemplary method, the sequential steps (b)-(e) are optionally repeated one or more times to increase the total thickness of the one or more metal oxide materials that enclose the solid core of the coated particles. In some embodiments, the reactor pressure is allowed to stabilize following step (a), step (b), and/or step (d). In some embodiments, the reactor contents are agitated prior to and/or during step (b), step (c), and/or step (e). In some embodiments, a subset of vapor or gaseous content is pumped out prior to step (c) and/or step (e).

The second exemplary method includes (e.g., consists of) the sequential steps of (a) loading the particles comprising the drug into a reactor, (b) reducing the reactor pressure to less than 1 Torr, (c) pressurizing the reactor to by adding a vaporous or gaseous metal precursor, (d) allowing the reactor pressure to stabilize, (e) agitating the reactor contents, (f) pumping out a subset of vapor or gaseous content and determining when to stop pumping based on analysis of content in reactor including metal precursor and byproduct of metal precursor reacting with exposed hydroxyl residues on substrate or on particle surface, (g) performing a sequence of pump-purge cycles of the reactor using insert gas, (h) pressuring the reactor by adding a vaporous or gaseous oxidant, (j) allowing the reactor pressure to stabilize, (k) agitating the reactor contents, (1) pumping out a subset of vapor or gaseous content and determining when to stop pumping based on analysis of content in reactor including metal precursor, byproduct of metal precursor reacting with exposed hydroxyl residues on substrate or on particle surface, and unreacted oxidant, and (m) performing a sequence of pump-purge cycles of the reactor using insert gas. In some embodiments of the second exemplary method, the sequential steps (b)-(m) are optionally repeated one or more times to increase the total thickness of the one or more metal oxide materials that enclose the solid core of the coated particles.

### Pharmaceutically acceptable excipients, diluents, and carriers

Pharmaceutically acceptable excipients include, but are not limited to:
(1) surfactants and polymers including: polyethylene glycol (PEG), polyvinylpyrrolidone (PVP), sodium lauryl sulfate, polyvinylalcohol, crospovidone, polyvinylpyrrolidonepolyvinylacrylate copolymer, cellulose derivatives, hydroxypropylmethyl cellulose, hydroxypropyl cellulose, carboxymethylethyl cellulose, hydroxypropyllmethyl cellulose phthalate, polyacrylates and polymethacrylates, urea, sugars, polyols, carbomer and their polymers, emulsifiers, sugar gum, starch, organic acids and their salts, vinyl pyrrolidone and vinyl acetate;
(2) binding agents such as cellulose, cross-linked polyvinylpyrrolidone, microcrystalline cellulose;
(3) filling agents such as lactose monohydrate, lactose anhydrous, microcrystalline cellulose and various starches;
(4) lubricating agents such as agents that act on the flowability of a powder to be compressed, including colloidal silicon dioxide, talc, stearic acid, magnesium stearate, calcium stearate, silica gel;
(5) sweeteners such as any natural or artificial sweetener including sucrose, xylitol, sodium saccharin, cyclamate, aspartame, and acesulfame K;
(6) flavoring agents;
(7) preservatives such as potassium sorbate, methylparaben, propylparaben, benzoic acid and its salts, other esters of parahydroxybenzoic acid such as butylparaben, alcohols such as ethyl or benzyl alcohol, phenolic chemicals such as phenol, or quarternary compounds such as benzalkonium chloride;
(8) buffers;
(9) Diluents such as pharmaceutically acceptable inert fillers, such as microcrystalline cellulose, lactose, dibasic calcium phosphate, saccharides, and/or mixtures of any of the foregoing;
(10) wetting agents such as corn starch, potato starch, maize starch, and modified starches, and mixtures thereof;
(11) disintegrants; such as croscarmellose sodium, crospovidone, sodium starch glycolate; and
(12) effervescent agents such as effervescent couples such as an organic acid (e.g., citric, tartaric, malic, fumaric, adipic, succinic, and alginic acids and anhydrides and acid salts), or a carbonate (e.g.,, sodium carbonate, potassium carbonate, magnesium carbonate, sodium glycine carbonate, L-lysine carbonate, and arginine carbonate) or bicarbonate (e.g. sodium bicarbonate or potassium bicarbonate)

### EXAMPLES

The following materials and methods were used in the Examples set forth herein.

### Example 1: Prepare particles comprising drug encapsulated by metal oxide and polymer layers

In this Example, one of the methods disclosed for preparing metal oxide and polymer encapsulated drugs is performed and the data is presented. In the ALD step for applying a metal oxide coating, the vaporous or gaseous metal precursor is tri-methyl aluminum (TMA), the byproduct gaseous methane is formed after TMA reacts with exposed hydroxyl groups on the particles or on surface of the coated particles, and the oxidant is water vapor. In the MLD step for applying a polymer coating, first adipoyl chloride is introduced and then ethylene diamine is introduced. A polyamide coating is formed.

### Method for ALD Coating

In brief, the method comprised the sequential steps of:
(a) loading particles comprising the drug into a reactor;
(b) reducing the reactor pressure to less than 1 Torr (e.g., below 50 mtorr);
(c) pressurizing the reactor to at least 1 Torr by adding a vaporous or gaseous TMA ;
(d) allowing the reactor pressure to stabilize;
(e) agitating the reactor contents;
(f) pumping out a subset of vapor or gaseous content, including gaseous methane and unreacted TMA, and determining when to stop pumping by performing RGA to monitor levels of gaseous methane and unreacted TMA in the reactor.
(g) performing a sequence of pump-purge cycles on the reactor using nitrogen gas;
(h) pressuring the reactor to at least 1 Torr by adding water vapor;
(i) allowing the reactor pressure to stabilize;
(j) agitating the reactor contents;
(k) pumping out a subset of vapor or gaseous content, including water vapor, and determining when to stop pumping by performing RGA to monitor levels of water vapor in the reactor;
(l) performing a sequence of pump-purge cycles on the reactor using nitrogen gas.
Additionally, the steps of (b)-(l) were repeated more than once to increase the total thickness of the aluminum oxide that enclose the drug particle core.

### Method for Hybrid MLD/ALD Coating

In brief, the method comprised the sequential steps of:
(a) loading particles comprising the drug into a reactor;
(b) reducing the reactor pressure to less than 1 Torr (e.g, below 50 mtorr);
(c) pressurizing the reactor to at least 1 Torr by adding a vaporous organic precursor, for example 1,2ethylanediol
(d) allowing the reactor pressure to stabilize;
(e) agitating the reactor contents;
(f) pumping out a subset of vapor or gaseous content, including gaseous unreacted 1,2-ethylenediol, and determining when to stop pumping by performing RGA to monitor levels of vaporous HCl and unreacted 1,2ethylenediol in the reactor.
(g) performing a sequence of pump-purge cycles on the reactor using nitrogen gas;
(h) pressuring the reactor to at least 1 Torr by adding vaporous metal oxide precursor, for example TMA;
(i) allowing the reactor pressure to stabilize;
(j) agitating the reactor contents;
(k) pumping out a subset of vapor or gaseous content, including methane, and determining when to stop pumping by performing RGA to monitor levels of methane vapor in the reactor;
(l) performing a sequence of pump-purge cycles on the reactor using nitrogen gas.
Additionally, the steps of (b)-(l) were repeated more than once to increase the total thickness of the hybrid layer that encloses the metal oxide coated core.

### Method for MLD Coating

In brief, the method comprised the sequential steps of:
(a) loading particles comprising the drug into a reactor;
(b) reducing the reactor pressure to less than 1 Torr (e.g., less than 50 mtorr);
(c) pressurizing the reactor to at least 1 Torr by adding a vaporous adipoyl chloride;
(d) allowing the reactor pressure to stabilize;
(e) agitating the reactor contents;
(f) pumping out a subset of vapor or gaseous content, including HCl vapor and unreacted adipoyl chloride, and determining when to stop pumping by performing RGA to monitor levels of gaseous HCl and unreacted adipoyl chloride in the reactor.
(g) performing a sequence of pump-purge cycles on the reactor using nitrogen gas;
(h) pressuring the reactor to at least 1 Torr by adding vaporous ethylene diamine;
(i) allowing the reactor pressure to stabilize;
(j) agitating the reactor contents;
(k) pumping out a subset of vapor or gaseous content, including HCl vapor, and determining when to stop pumping by performing RGA to monitor levels of HCl vapor in the reactor; and
(l) performing a sequence of pump-purge cycles on the reactor using nitrogen gas.

Additionally, the steps of (b)-(l) were repeated more than once to increase the total thickness of the polymer that encloses the metal oxide coated core.

FIG. 2 includes representative process conditions for performing MLD deposition of a polymer layer (polyamide).

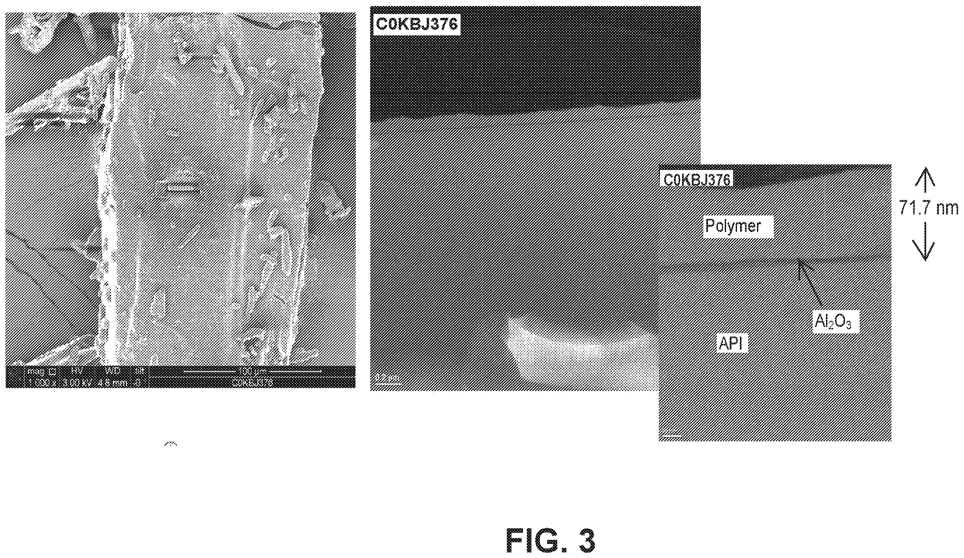

FIG. 3 is an SEM image (left side) of an API particle coated essentially as described above and a TEM cross (right side and enlarged at far right) section of an API particle coated essentially as described above.

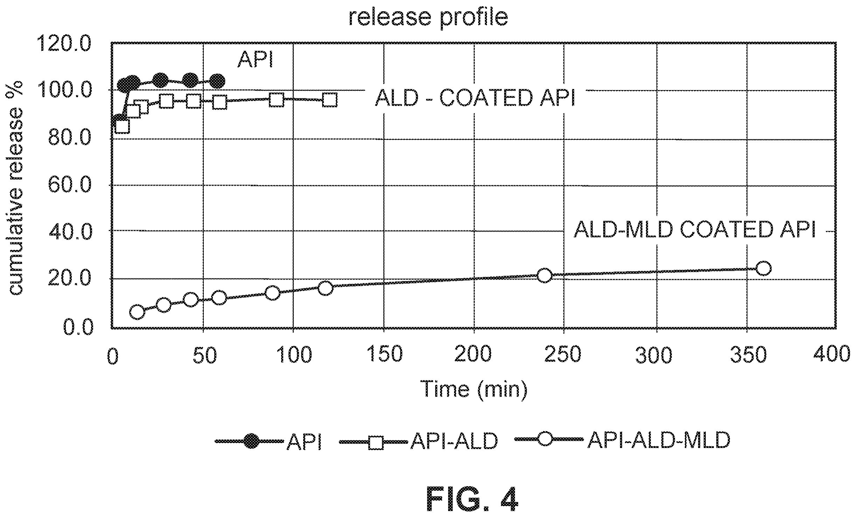

As can be seem in FIG. 4, a combination of ALD and MLD coating can greatly reduce the dissolution rate of particles of acetaminophen. Here the dissolution was tested in phosphate buffer, pH 5.8, 50 rpm, 325 mg API in 900ml media.

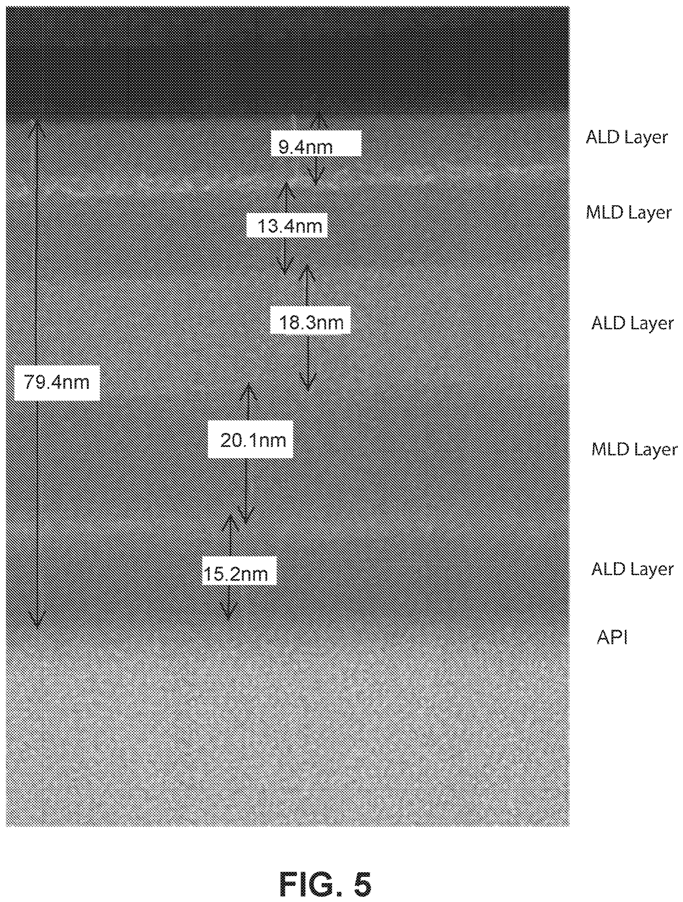

FIG. 5 is a TEM image of a cross-section of an API particle coated with alternating metal oxide and polyamide coating layers. In each case 10-20 cycles were performed.

In the following, implementations of embodiments of the invention will be explained:
1. A pharmaceutical composition comprising individual particles comprising an API-containing core enclosed by an inner metal oxide layer and polymer layer adjacent the metal oxide layer, wherein the core has a median particle size, on a volume average basis, between 0.1 µm and 20 µm, the inner metal oxide layer has an average thickness of 0.1 nm to 30 nm and the polymer layer has an average thickness of 0.1 nm to 400 nm.
2. The pharmaceutical composition of implementation 1, wherein the inner metal oxide layer conforms the core and is substantially pin-hole free and the outer polymer layer conforms to the inner metal oxide layer and is substantially pin-hole free.
3. The pharmaceutical composition of implementation 1, wherein the core has a median particle size, on a volume average basis between 0.1 µm and 10 µm.
4. The pharmaceutical composition of any of the forgoing implementations, wherein the core further comprises one or more pharmaceutically acceptable excipients.
5. The pharmaceutical composition of any of the forgoing implementations, wherein the core comprises a first API and a second API.
6. The pharmaceutical composition of any of implementations 1-4, wherein the core consists of an API.
7. The pharmaceutical composition of any of implementations 1-4, wherein the core comprises a first API and a second API.
8. The pharmaceutical composition of any of implementations 1-7, wherein the particles comprise an outer metal oxide layer adjacent the polymer layer.
9. The pharmaceutical composition of any of the forgoing implementations, wherein the metal oxide layer comprises aluminum oxide, titanium oxide, iron oxide, silicon dioxide, magnesium oxide or zinc oxide.
10. The pharmaceutical composition of any of the forgoing implementations, wherein the polymer layer comprises a polyamide, a polyimide, a polyurea, a polyurethane, a polythiourea, a polyester or a polyimine.
11. A pharmaceutical composition comprising coated particles comprising an API-containing core enclosed by one or more metal oxide layers and one or more polymer layers, prepared by a method comprising the sequential steps of:
   (a) performing atomic layer deposition to apply a metal oxide layer to particles comprising an API thereby preparing particles comprising an API enclosed by a metal oxide layer; and subsequently
   (b) performing molecular layer deposition to apply a polymer layer to the particles comprising a drug enclosed by a metal oxide layers, thereby preparing coated particles comprising an API-containing core enclosed by one or more metal oxide layers and one or more polymer layers.
12. The pharmaceutical composition of implementation 11, wherein the step of performing atomic layer deposition comprises:
   (a1) loading the particles comprising the API into a reactor;
   (a2) applying a vaporous or gaseous metal precursor to the particles in the reactor;
   (a3) performing one or more pump-purge cycles of the reactor using inert gas;
   (a4) applying a vaporous or gaseous oxidant to the particles in the reactor; and
   (a5) performing one or more pump-purge cycles of the reactor using inert gas.
13. The pharmaceutical composition of implementation 12, wherein steps (a2) - (a5) are performed two or more times to increase the total thickness of the metal oxide layer before step (b) is performed.
14. The pharmaceutical composition of implementation 13, wherein steps (a2) - (a5) are performed 5 - 20 times to increase the total thickness of the metal oxide layer before step (b) is performed.
15. The pharmaceutical composition of any of implementations 11-14, wherein the step of performing molecular layer deposition comprises:
   (b1) applying vaporous polymer precursor A to the particles comprising an API containing enclosed by a oxide layer in the reactor;
   (b2) performing one or more pump-purge cycles of the reactor using inert gas;
   (b3) applying vaporous polymer precursor B to the particles in the reactor; and
   (b4) performing one or more pump-purge cycles of the reactor using inert gas.
16. The pharmaceutical composition of implementation 14, wherein steps (b1) - (b4) are performed two or more times to increase the total thickness of the polymer layer.
17. The pharmaceutical composition of implementation 16, wherein steps (a2) - (a5) are performed 5 - 20 times to increase the total thickness of the metal oxide layer before step (b) is performed.
18. The pharmaceutical composition of implementation 11, wherein the reactor contents are agitated prior to and/or during step (a) and/or step (b).
19. The pharmaceutical composition of implementation 12, wherein the reactor pressure is allowed to stabilize following step (a1), step (a2), and/or step (a4).
20. The pharmaceutical composition of implementation 11, wherein the reactor contents are agitated prior to and/or during step (a1), step (a3), and/or step (a5).
21. The pharmaceutical composition of implementation 12, wherein a subset of vapor or gaseous content is pumped out prior to step (a3) and/or step (a5).
22. The pharmaceutical composition of implementation 11, wherein the metal oxide layer has a thickness in range of 0.1 nm to 30 nm.
23. The pharmaceutical composition of implementation 11, wherein the polymer layer has a thickness in range of 0.1 nm to 100 nm.
24. The pharmaceutical composition of implementation 21, wherein steps (a2) - (a5) are repeated two or more times and the particles are not removed from the reactor between each repetition.
25. The pharmaceutical composition of implementation 11, wherein steps (a2) - (a5) are repeated two or more times and the particles are not removed from the reactor during step (a).
26. The pharmaceutical composition of implementation 11, wherein steps (b1) - (b4) are repeated two or more times and the particles are not removed from the reactor between each repetition.
27. The pharmaceutical composition of implementation 11, wherein steps (b1) - (b4) are repeated two or more times and the particles are not removed from the reactor during step (b).
28. The pharmaceutical composition of implementation 11, wherein the particles comprising an API further comprise one or more pharmaceutically acceptable excipients.
29. The pharmaceutical composition of implementation 11, wherein the particles comprising an API have a median particle size, on a volume average basis between 0.1 µm and 20 µm prior to step (a).
30. The pharmaceutical composition of implementation 11, wherein the particles subjected to step (a) comprise a first API and a second API.
31. The pharmaceutical composition of implementation 11, wherein the particles subjected to step (a) consist of an API.
32. The pharmaceutical composition of implementation 11, wherein the particles subjected to step (a) consist of a first API and a second API.
33. The pharmaceutical composition of implementation 11, wherein the particles subjected to step (a) comprise an API and one or more pharmaceutically acceptable excipients.
34. The pharmaceutical composition of implementation 11, wherein the particles subjected to step (a) comprise a first API, a second API and one or more pharmaceutically acceptable excipients.
35. The pharmaceutical composition of implementation 11, wherein the particles subjected to step (a) consist of an API and one or more pharmaceutically acceptable excipients.
36. The pharmaceutical composition of implementation 11, wherein the particles subjected to step (a) consist of a first API, a second API and one or more pharmaceutically acceptable excipients.
37. The pharmaceutical composition of any of the forgoing implementations, wherein the metal oxide layer comprises aluminum oxide, titanium oxide, iron oxide, silicon dioxide, magnesium oxide or zinc oxide.
38. The pharmaceutical composition of any of the forgoing implementations, wherein the polymer layer comprises a polyamide, a polyimide, a polyurea, a polyurethane, a polythiourea, a polyester or a polyimine.
39. The pharmaceutical composition of any of the forgoing implementations, wherein the polymer layer is biodegradable.
40. The pharmaceutical composition of any of the forgoing implementations comprising an inner metal oxide layer, an intermediate polymer layer and an out metal oxide layer.
41. A method of preparing coated particles comprising an active pharmaceutical ingredient (API)-containing core enclosed by one or more metal oxide layers and one or more polymer layers, the method comprising the sequential steps of:
   (a) performing atomic layer deposition to apply a metal oxide layer to particles comprising an API thereby preparing particles comprising an API enclosed by a metal oxide layer; and subsequently
   (b) performing molecular layer deposition to apply a polymer layer to the particles comprising a drug enclosed by a metal oxide layer, thereby preparing coated particles comprising an API-containing core enclosed by one more metal oxide layers and one or more polymer layers.
42. The method of implementation 41, wherein the step of performing atomic layer deposition comprises:
   (a1) loading the particles comprising the API into a reactor;
   (a2) applying a vaporous or gaseous metal precursor to the particles in the reactor;
   (a3) performing one or more pump-purge cycles of the reactor using inert gas;
   (a4) applying a vaporous or gaseous oxidant to the particles in the reactor; and
   (a5) performing one or more pump-purge cycles of the reactor using inert gas.
43. The method of implementation 42, wherein steps (a2) - (a5) are performed two or more times to increase the total thickness of the metal oxide layer before step (b) is performed.
44. The method of implementation 43, wherein steps (a2) - (a5) are performed 5 - 50 times to increase the total thickness of the metal oxide layer before step (b) is performed.
45. The method of any of implementations 41-44, wherein the step of performing molecular layer deposition comprises:
   (b1) applying vaporous polymer precursor A to the particles comprising an API containing enclosed by a metal oxide layer in the reactor;
   (b2) performing one or more pump-purge cycles of the reactor using inert gas;
   (b3) applying vaporous polymer precursor B to the particles in the reactor; and
   (b4) performing one or more pump-purge cycles of the reactor using inert gas.
46. The method of implementation 44, wherein steps (b1) - (b4) are performed two or more times to increase the total thickness of the polymer layer.
47. The method of implementation 46, wherein steps (a2) - (a5) are performed 5 - 50 times to increase the total thickness of the metal oxide layer before step (b) is performed.
48. The method of implementation 41, wherein the reactor contents are agitated prior to and/or during step (a) and/or step (b).
49. The method of implementation 42, wherein the reactor pressure is allowed to stabilize following step (a1), step (a2), and/or step (a4).
50. The method of implementation 41, wherein the reactor contents are agitated prior to and/or during step (a1), step (a3), and/or step (a5).
51. The method of implementation 42, wherein a subset of vapor or gaseous content is pumped out prior to step (a3) and/or step (a5).
52. The method of implementation 41, wherein the metal oxide layer has a thickness in range of 0.1 nm to 20 nm.
53. The method of implementation 41, wherein the polymer layer has a thickness in range of 0.1 nm to 400 nm.
54. The method of implementation 41, wherein steps (a2) - (a5) are repeated two or more times and the particles are not removed from the reactor between each repetition.
55. The method of implementation 41, wherein steps (a2) - (a5) are repeated two or more times and the particles are not removed from the reactor during step (a).
56. The method of implementation 41, wherein steps (b1) - (b4) are repeated two or more times and the particles are not removed from the reactor between each repetition.
57. The method of implementation 41, wherein steps (b1) - (b4) are repeated two or more times and the particles are not removed from the reactor during step (b)
58. The method of implementation 41, wherein the particles comprising an API further comprise one or more pharmaceutically acceptable excipients.
59. The method of implementation 41, wherein the particles comprising an API have a median particle size, on a volume average basis between 0.1 µm and 20 µm prior to step (a).
60. The method of implementation 41, wherein the particles have a median particle size, on a volume average basis between 0.1 µm and 5 µm prior to step (a).
61. The method of implementation 41, wherein the particles have a median particle size, on a volume average basis between 0.1 µm and 1000 µm prior to step (a).
62. The method of implementation 41, wherein the particles subjected to step (a) comprise a first API and a second API.
63. The method of implementation 41, wherein the particles subjected to step (a) consist of an API.
64. The method of implementation 41, wherein the particles subjected to step (a) consist of a first API and a second API.
65. The method of implementation 41, wherein the particles subjected to step (a) comprise an API and one or more pharmaceutically acceptable excipients.
66. The method of implementation 41, wherein the particles subjected to step (a) comprise a first API, a second API and one or more pharmaceutically acceptable excipients.
67. The method of implementation 41, wherein the particles subjected to step (a) consist of an API and one or more pharmaceutically acceptable excipients.
68. The method of implementation 41, wherein the particles subjected to step (a) consist of a first API, a second API and one or more pharmaceutically acceptable excipients.
69. The method of implementation 41, further comprising admixing the coated particles comprising an API-containing core enclosed by one or more metal oxide layers and one or more polymer layers with a pharmaceutically acceptable diluent or carrier.
70. The method of implementation 41, further comprising processing the coated particles comprising an API-containing core enclosed by one or more metal oxide layers and one or more polymer layers to form a tablet or capsule.
71. The method of implementation 41, further comprising admixing the coated particles comprising an API-containing core enclosed by one or more metal oxide layers and one or more polymer layers with a pharmaceutically acceptable diluent or carrier to form a mixture a processing the mixture to form a table or capsule.
72. The method of implementation 41, further comprising: performing atomic layer deposition to apply a metal oxide layer to the particles comprising an API-containing core enclosed by a metal oxide layer and a polymer layer thereby preparing particles comprising an API enclosed by an inner metal oxide layer, an intermediate polymer layer and an outer metal oxide layer.
73. The method of implementation 42, further comprising
   performing molecular layer deposition to apply a polymer layer to the particles comprising an API enclosed by an inner metal oxide layer, an intermediate polymer layer and an outer metal oxide layer thereby preparing particles comprising an API enclosed by an inner metal oxide layer, an inner intermediate polymer layer and an outer intermediate metal oxide layer and an outer polymer layer.
74. The method of any of implementations 41-73, further comprising admixing the coated particles comprising an API-containing core enclosed by one or more metal oxide layers and one or more polymer layers with a pharmaceutically acceptable diluent or carrier.
75. The method of any of implementations 41-73, further comprising processing the coated particles comprising an API-containing core enclosed by one or more metal oxide layers and one or more polymer layers to form a tablet or capsule.
76. The method of any of implementations 41-73, further comprising admixing the coated particles comprising an API-containing core enclosed by one or more metal oxide layers and one or more polymer layers with a pharmaceutically acceptable diluent or carrier to form a mixture a processing the mixture to form a table or capsule.

## Claims

1. A pharmaceutical composition comprising individual particles comprising an active pharmaceutical ingredient, API, containing core enclosed by an inner metal oxide layer and an outer polymer layer adjacent the metal oxide layer.

2. The pharmaceutical composition of claim 1, wherein the core has a median particle size, on a volume average basis, between 0.1 µm and 20 µm.

3. The pharmaceutical composition of any of the forgoing claims, wherein the inner metal oxide layer has an average thickness of 0.1 nm to 30 nm.

4. The pharmaceutical composition of any of the forgoing claims, wherein the outer polymer layer has an average thickness of 0.1 nm to 400 nm.

5. The pharmaceutical composition of any of the forgoing claims, wherein the inner metal oxide layer conforms to the core and is substantially pin-hole free.

6. The pharmaceutical composition of any of the forgoing claims, wherein the outer polymer layer conforms to the inner metal oxide layer and is substantially pin-hole free.

7. The pharmaceutical composition of any of the forgoing claims, wherein the metal oxide layer comprises aluminum oxide, titanium oxide, silicon dioxide, or zinc oxide.

8. The pharmaceutical composition of any of the forgoing claims, wherein the core further comprises one or more pharmaceutically acceptable excipients.

9. The pharmaceutical composition of any of the forgoing claims, wherein the polymer layer comprises a polyamide, a polyimide, a polyurea, a polyurethane, a polythiourea, a polyester, or a polyimine.

10. A method of preparing coated particles, the method comprising the sequential steps of:
a) applying a metal oxide layer to particles (50) comprising an API thereby preparing particles comprising an API enclosed by a metal oxide layer; and subsequently
b) applying a polymer layer to the particles comprising a drug enclosed by a metal oxide layer.

11. The method of claim 10, wherein the coated comprising an API-containing core enclosed by one more metal oxide layers and one or more polymer layers.

12. The method of claim 10, wherein step (a) comprises:
a1) loading the particles (50) comprising the API into a reactor (10);
a2) applying a vaporous or gaseous metal precursor to the particles (50) in the reactor (10);
a3) performing one or more pump-purge cycles of the reactor (10) using inert gas;
a4) applying a vaporous or gaseous oxidant to the particles (50) in the reactor (10); and
a5) performing one or more pump-purge cycles of the reactor (10) using inert gas.

13. The method of any of claims 10-12, wherein step (b) comprises:
b1) applying vaporous polymer precursor A to the particles (50) comprising an API containing enclosed by a metal oxide layer in the reactor (10);
b2) performing one or more pump-purge cycles of the reactor (10) using inert gas;
b3) applying vaporous polymer precursor B to the particles (50) in the reactor (10); and
b4) performing one or more pump-purge cycles of the reactor using inert gas (10).

14. The method of claim 10, wherein the metal oxide layer has a thickness in range of 0.1 nm to 20 nm.

15. The method of claim 10, wherein the polymer layer has a thickness in range of 0.1 nm to 400 nm.
